# EUROPÄISCHE PATENTSCHRIFT

(11) **EP 0 538 900 B1**
(45) Veröffentlichungstag und Bekanntmachung des Hinweises auf die Patenterteilung: **05.03.1997**
(21) Anmeldenummer: 92118226.7
(22) Anmeldetag: 24.10.1992
(51) Int. Cl.: C09B 62/51

(54) **Wasserlösliche Azoverbindungen, Verfahren zu deren Herstellung und ihre Verwendung als Farbstoffe**
Water-soluble azo compounds, process for their preparation and their use as dyes
Composés azoiques solubles dans l'eau, procédé pour leur préparation et leur utilisation comme colorants

(30) Priorität: 24.10.1991 DE 4135067
(43) Veröffentlichungstag der Anmeldung: 28.04.1993
(73) Patentinhaber: HOECHST AKTIENGESELLSCHAFT, 65926 Frankfurt am Main (DE)
(72) Erfinder: Schläfer, Ludwig, Dr., W-6233 Kelkheim/Ts (DE); Russ, Werner Hubert, Dr., W-6093 Flörsheim/M. (DE)

(56) Entgegenhaltungen:
- EP-A- 0 036 383
- US-A- 3 518 245

## Beschreibung

Die Erfindung liegt auf dem technischen Gebiet der faserreaktiven Azofarbstoffe.

Mit der vorliegenden Erfindung wurden neue, wertvolle faserreaktive Monoazoverbindungen mit guten bis sehr guten Naßechtheitseigenschaften der mit ihnen erhältlichen Färbungen gefunden, die sich sehr gut für den Ätz- und Reservedruck eignen und somit in breiterem Anwendungsumfang zum Färben von Fasermaterialien einsetzen lassen. Beim Ätzdruck wird auf eine gefärbte Stoffbahn (Fond) in einem gewünschten Muster ein Ätzmittel aufgedruckt; das Ätzmittel zerstört den Farbstoff, sofern der Farbstoff ätzbar ist, so daß nach Fertigstellung des Ätzverfahrens ein weißes Muster auf der Färbung erscheint (Weißätze). Enthält die aufgedruckte Ätze zusätzlich einen ätzestabilen Farbstoff, so erhält man nach Fertigstellung des Ätzvorgangs und üblicher Behandlung zur Fixierung dieses beigegebenen Farbstoffes auf dem Fond ein Druckmuster in einem anderen Farbton (Buntätze). Bei dem Reservedruck wird zunächst das Gewebe mit einem geeigneten Reservierungsmittel in einem gewünschten Muster bedruckt. Das so vorbedruckte Gewebe wird anschließend mit einem Farbstoff, der dafür geeignet ist, sich mit dem Reservierungsmittel zu verbinden und somit nicht mehr in der Lage ist, auf dem Gewebe zu fixieren, überfärbt (überklotzt oder überdruckt) mit der Folge, daß an den reservierten Stellen keine Farbstoffixierung eintritt und somit die erhaltene Färbung ein weißes Muster gemäß dem Muster des Reservierungsmittels besitzt.

Die neuen, erfindungsgemäßen Monoazoverbindungen besitzen die allgemeinen Formel (1) 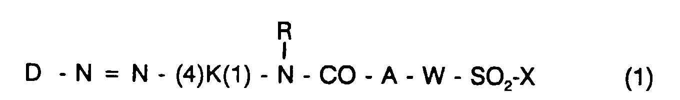 in welcher bedeuten:
- D: ist Monosulfophenyl oder Disulfophenyl, die beide durch 1 oder 2 Substituenten aus der Gruppe Alkyl von 1 bis 4 C-Atomen, wie Ethyl und insbesondere Methyl, Alkoxy von 1 bis 4 C-Atomen, wie Ethoxy und insbesondere Methoxy, Halogen, wie Brom und insbesondere Chlor, Carboxy und Alkanoylamino von 2 bis 5 C-Atomen, wie Propionylamino und insbesondere Acetylamino, substituiert sein können, oder ist Mono-, Di- oder Trisulfo-naphthyl;
- K: ist als Rest einer Kupplungskomponente der para-Phenylenrest, der unsubstituiert oder durch 1 oder 2 Substituenten aus der Gruppe Alkyl von 1 bis 4 C-Atomen, wie Ethyl und insbesondere Methyl, Alkoxy von 1 bis 4 C-Atomen, wie Ethoxy und insbesondere Methoxy, Halogen, wie Chlor, Alkanoylamino von 2 bis 5 C-Atomen, wie Propionylamino und Acetylamino, Ureido, Carbamoyl und Sulfo substituiert ist, oder ist der 1,4-Naphthylen-Rest, der durch 1 oder 2 Sulfogruppen und/oder 1 Alkoxygruppe von 1 bis 4 C-Atomen, wie Ethoxy und insbesondere Methoxy, substituiert sein kann;
- R: ist Wasserstoff oder Alkyl von 1 bis 4 C-Atomen, wie Ethyl und insbesondere Methyl, bevorzugt Wasserstoff;
- A: ist Phenylen, wie para-Phenylen und insbesondere meta-Phenylen, oder eine direkte Bindung;
- W: ist eine direkte Bindung oder Alkylen von 1 bis 4 C-Atomen, wie Ethylen und Methylen, wobei jedoch A und W nicht gleichzeitig eine direkte Bindung sind;
- X: ist Vinyl oder ist Ethyl, das in β-Stellung durch einen Substituenten substituiert ist, der durch Alkali unter Bildung der Vinylgruppe eliminierbar ist.

β-Ständige, alkalisch eliminierbare Substituenten in der Ethylgruppe von X sind beispielsweise Alkanoyloxy von 2 bis 5 C-Atomen, wie Acetyloxy, Benzoyloxy, Sulfobenzoyloxy, Toluolsulfonyloxy, Halogen, wie Brom und insbesondere Chlor, Dimethylamino, Diethylamino, Phosphato, Thiosulfato und Sulfato.

Die Gruppen "Sulfo", "Carboxy", "Phosphato", "Thiosulfato" und "Sulfato" schließen sowohl deren Säureform als auch deren Salzform ein. Demgemäß bedeuten Sulfogruppen Gruppen entsprechend der allgemeinen Formel -SO₃M, Carboxygruppen Gruppen entsprechend der allgemeinen Formel -COOM, Phosphatogruppen Gruppen entsprechend der allgemeinen Formel -OPO₃M₂, Thiosulfatogruppen Gruppen entsprechend der allgemeinen Formel -S-SO₃M und Sulfatogruppen Gruppen entsprechend der allgemeinen Formel -OSO₃M, jeweils mit M der obengenannten Bedeutung.

Sofern D ein Monosulfophenyl ist, ist dieses bevorzugt durch 1 oder 2, bevorzugt 1, Substituenten aus der Gruppe Alkyl von 1 bis 4 C-Atomen und Alkoxy von 1 bis 4 C-Atomen substituiert. Außer solchen gegebenenfalls substituierten Monosulfophenylresten ist der Disulfophenyl- und der Di- und Trisulfo-naphthyl-Rest bevorzugt, wobei der Naphthylrest bevorzugt ein Naphth-2-yl-Rest ist.

Bevorzugt ist K 1,4-Phenylen, das durch 1 Substituenten, wie bspw. Methyl, Ethyl, Methoxy, Ethoxy, Acetylamino, Propionylamino, Ureido oder Sulfo substituiert sein kann, oder ist Naphthylen, das durch Sulfo, Methoxy, Ethoxy, Methyl oder Ethyl substituiert sein kann. Insbesondere bevorzugt ist K der 3-Ureido-1,4-phenylen- oder der 8-Sulfo-1,4-naphthylen-Rest ist.

Von den erfindungsgemäßen Verbindungen der allgemeinen Formel (1) sind weiterhin diejenigen bevorzugt, in welchen X für Vinyl, β-Chlorethyl oder β-Sulfatoethyl, insbesondere bevorzugt für Vinyl oder β-Chlorethyl, steht.

Reste D in den Verbindungen der allgemeinen Formel (1) sind beispielsweise 2,4-Disulfo-phenyl, 2,5-Disulfo-4-methoxy-phenyl, 4-Sulfo-2-methoxy-phenyl, 4-Sulfo-2,5-dimethoxy-phenyl, 2,4-Disulfo-naphth-1-yl, 2,5-Disulfo-naphth-1-yl, 2,5,7-Trisulfo-naphth-1-yl, 2,4,7-Trisulfo-naphth-1-yl, 3,5,7-Trisulfo-naphth-1-yl, 3,6,8-Trisulfo-naphth-1-yl, 3,6-Disulfo-naphth-1-yl, 4,7-Disulfo-naphth-1-yl, 4,6-Disulfo-naphth-1-yl, 4,8-Disulfo-naphth-2-yl, 4,6,8-Trisulfo-naphth-2-yl und 3,6,8-Trisulfo-naphth-2-yl.

Reste der allgemeinen Formel -(4)K(1)-N(R)- in den Verbindungen der allgemeinen Formel (1) sind beispielsweise 3-Methyl-1,4-phen-1,4-ylen-1-amino, Phen-1,4-ylen-1-(N-methyl)-amino, Phen-1,4-ylen-1-amino, 2,5-Dimethyl-phen-1,4-ylen-1-amino, 2-Methoxy-5-methyl-phen-1,4-ylen-1-amino, 3-Acetylamino-phen-1,4-ylen-1-amino, 3-Ureido-phen-1,4-ylen-1-amino, Naphth-1,4-ylen-1-amino, 6-Sulfo-naphth-1,4-ylen-1-amino, 7-Sulfo-naphth-1,4-ylen-1-amino und 8-Sulfo-naphth-1,4-ylen-1-amino.

Reste entsprechend der allgemeinen Formel -A-W-SO₂-X in den Verbindungen der allgemeinen Formel (1) sind beispielsweise 3-(β-Chlorethylsulfonyl)-phenyl, 3-(Vinylsulfonyl)-phenyl, 4-(β-Chlorethylsulfonyl-methyl)-phenyl, β-(β'-Chlorethylsulfonyl)-ethyl und γ-(β'-Chlorethylsulfonyl)-propyl.

Die vorliegende Erfindung betrifft weiterhin Verfahren zur Herstellung der erfindungsgemäßen Azoverbindungen der allgemeinen Formel (1), die dadurch gekennzeichnet sind, daß man eine Amino-Azoverbindung der allgemeinen Formel (2)

D - N = N - K - N(R) - H (2)

in welcher D, K und R die obengenannten Bedeutungen haben, mit einer Verbindung der allgemeinen Formel (3)

Hal - CO - A - W - SO₂-X (3)

in welcher Hal für Brom oder bevorzugt Chlor steht und A, W und X die obengenannten Bedeutungen haben, bei einer Temperatur zwischen 0 und 40° C, bevorzugt zwischen 15 und 30°C, und bei einem pH-Wert zwischen 1 und 9, bevorzugt zwischen 6 und 8, umsetzt.

Sämtliche Ausgangsverbindungen, so die Diazokomponenten der allgemeinen Formel D-NH₂, die Kupplungskomponenten der Formel H-K-N(R)-H und die Säurechloride der allgemeinen Formel (3) sind in der Literatur bekannt.

Ausgangsverbindungen der allgemeinen Formel D-NH₂, die als Diazokomponenten zur Synthese der Ausgangs-Azoverbindungen der allgemeinen Formel (2) dienen können, sind beispielsweise 2,4-Disulfo-anilin, 2,5-Disulfo-anilin, 2,5-Disulfo-4-methyl-anilin, 2,5-Disulfo-4-methoxy-anilin, 4-Sulfo-2-methoxy-anilin, 4-Sulfo-2,5-dimethoxy-anilin, 2,4-Disulfo-1-aminonaphthalin, 2,5-Disulfo-1-aminonaphthalin, 2,5,7-Trisulfo-1-aminonaphthalin, 2,4,7-Trisulfo-1-aminonaphthalin, 3,5,7-Trisulfo-1-aminonaphthalin, 3,6,8-Trisulfo-1-aminonaphthalin, 3,6-Disulfo-4,7-Disulfo-1-aminonaphthalin, 4,7-Disulfo-1-aminonaphthalin, 4,6-Disulfo-1-aminonaphthalin, 4,8-Disulfo-2-aminonaphthalin, 4,6,8-Trisulfo-2-aminonaphthalin und 3,6,8-Trisulfo-2-aminonaphthalin.

Kupplungskomponenten sind beispielsweise Anilin, N-Methyl-anilin, 3-Methyl-anilin, 2,5-Dimethyl-anilin, 2-Methoxy-5-methyl-anilin, 3-Acetylamino-anilin, 3-Ureido-anilin, 1-Amino-naphthalin, 6-Sulfo-1-amino-naphthalin, 7-Sulfo-1-aminonaphthalin, 8-Sulfo-1-aminonaphthalin und 2-Methoxy-6-sulfo-1-aminonaphthalin.

Ausgangsverbindungen der allgemeinen Formel (3) sind beispielsweise 3-(β-Chlorethylsulfonyl)-benzoesäurechlorid, 4-(β-Chlorethylsulfonyl-methyl)-benzoesäurechlorid, 2-(β-Chlorethylsulfonyl)-priopionsäurechlorid und 3-(β-Chlorethylsulfonyl)-butansäurechlorid.

Die Umsetzung der Verbindungen der allgemeinen Formel (2) und (3) erfolgt in wäßrig-organischem oder bevorzugt wäßrigem Medium, wobei, sofern die Umsetzung in einem wäßrig-organischen Medium durchgeführt wird, das organische Medium ein gegenüber den Reaktanten inertes und, bevorzugt, mit Wasser mischbares Lösemittel ist; solche Lösungsmittel sind beispielsweise Toluol und insbesondere Aceton, N-Methyl-pyrrolidon und Dimethylsulfoxid.

Die Abscheidung der erfindungsgemäß hergestellten Verbindungen aus den Syntheseansätzen erfolgt nach allgemein bekannten Methoden, so beispielsweise durch Ausfällen aus dem Reaktionsmedium mittels einem Elektrolyten, wie beispielsweise Natriumchlorid und Kaliumchlorid, oder durch Eindampfen oder Sprühtrocknung der Reaktionslösung, wobei der Reaktionslösung eine Puffersubstanz zugefügt werden kann. Die neuen erfindungsgemäßen Azoverbindungen der allgemeinen Formel (1) - im nachfolgenden Verbindungen (1) genannt - haben faserreaktive Eigenschaften und besitzen sehr gute Farbstoffeigenschaften. Sie können deshalb zum Färben von hydroxy- und/oder carbonamidgruppenhaltigem Material, insbesondere Fasermaterial, wie auch von Leder, verwendet werden. Ebenso können die bei der Synthese der Verbindungen (1) anfallenden Lösungen, gegebenenfalls nach Zusatz einer üblichen Puffersubstanz, die einen pH-Wert zwischen 3 und 7 einzuhalten vermag, und gegebenenfalls nach Konzentrierung der Lösung, direkt als Flüssigpräparation der färberischen Verwendung zugeführt werden.

Gegenstand der vorliegenden Erfindung ist deshalb auch die Verwendung der erfindungsgemäßen Verbindungen (1) zum Färben (einschließlich Bedrucken) von hydroxy- und/oder carbonamidgruppenhaltigen Materialien, insbesondere Fasermaterialien, bzw. Verfahren zu deren Anwendung auf diesen Substraten. Hierbei kann man analog bekannten Verfahrensweisen vorgehen.

Hydroxygrupppenhaltige Materialien sind solche natürlichen oder synthetischen Ursprungs, wie beispielsweise Cellulosefasern oder deren Regeneratprodukte und Polyvinylalkohole oder cellulosehaltige Produkte, wie Papier.

Cellulosefasermaterialien sind vorzugsweise Baumwolle, aber auch andere Pflanzenfasern, wie Leinen, Hanf, Jute und Ramiefasern; regenerierte Cellulosefasern sind beispielsweise Zellwolle und Viskosekunstseide.

Carbonamidgruppenhaltige Materialien sind beispielsweise synthetische und natürliche Polyamide und Polyurethane sowohl in Form der Masse, wie beispielsweise Folien, als auch in Form von Fasern, beispielsweise Wolle und andere Tierhaare, Seide, Leder, Polyamid-6,6, Polyamid-6, Polyamid-11 und Polyamid-4.

Die Verbindungen (1) lassen sich, gemäß der erfindungsgemäßen Anwendung, auf den genannten Substraten nach den für wasserlösliche Farbstoffe, insbesondere faserreaktive Farbstoffe, bekannten Anwendungstechniken applizieren und fixieren, so beispielsweise, indem man die Verbindung (1) in gelöster Form auf das Substrat aufbringt oder sie darin einbringt und sie auf diesem oder in diesem durch Hitzeeinwirkung oder durch Einwirkung eines alkalisch wirkenden Mittels oder durch beide Maßnahmen, fixiert. Solche Färbe- und Fixierweisen sind sowohl in der Fachliteratur als auch in der Patentliteratur zahlreich beschrieben, wie beispielsweise in der Europäischen Patentanmeldungs-Veröffentlichung Nr. 0 218 131.

Die erfindungsgemäßen Verbindungen (1) besitzen eine gute bis sehr gute Löslichkeit in Wasser und eine gute Stabilität in den Druckpasten und Färbebädern. Sie zeichnen sich durch hohe Farbstärke und guten Farbaufbau aus und liefern mit sehr hohen Fixierausbeuten farbstarke, gelbe Färbungen und Drucke, die sich durch gute Gebrauchs- und Fabrikationsechtheiten, wie beispielsweise Wasch-, Meerwasser-, Wasser-, Chlorbadewasser-, Säure-, Alkali-, Überfärbe-, Schweiß-, Abgas-, Bügel-, Plissier-, Dekatier-, Trockenreinigungs- und Reibechtheiten, auszeichnen. Die Lichtechtheiten der erhaltenen Färbungen und Drucke sowohl in trockenem als auch in mit Trinkwasser oder in mit einer sauren oder alkalischen Schweißlösung feuchten Zustand sind sehr gut. Nicht fixierte Farbstoffanteile lassen sich gut auswaschen. Im Druck auf Cellulosefasermaterialien erhält man scharfe Konturen mit einem klaren Weißfond. Drucke und Färbungen flecken in nicht fixiertem Zustand beim Ablegen nicht ab bzw. bluten nicht aus.

Insbesondere eignen sich die erfindungsgemäßen Verbindungen (1) jedoch für den Ätz- und Reservedruck. Ihre Verwendbarkeit in diesen beiden technisch bedeutenden Anwendungsverfahren beruht darauf, daß Färbungen dieser neuen Verbindungen (1) rein weiß ätzbar sind bzw. daß die neuen Verbindungen (1) Cellulosefasermaterial an den Stellen, an denen es mit einem Reservierungsmittel bedruckt oder imprägniert wurde, auch unter den üblichen Fixierbedingungen für faserreaktive Farbstoffe, d. h. in Gegenwart von alkalisch wirkenden Mitteln und höheren Temperaturen, wie erwünscht, nicht anfärben, so daß die erfindungsgemäßen Verbindungen (1) sich problemlos für die Herstellung von Färbungen eignen, deren Negativmuster durch ein Reservierungsmittel vorgegeben ist.

Die nachfolgenden Beispiele dienen zur Erläuterung der Erfindung. Die Teile sind Gewichtsteile, die Prozentangaben stellen Gewichtsprozente dar, sofern nicht anders vermerkt. Gewichtsteile beziehen sich zu Volumenteilen wie Kilogramm zu Liter.

Die in diesen Beispielen formelmäßig beschriebenen Verbindungen sind in Form der freien Säuren angegeben; im allgemeinen werden sie in Form ihrer Alkalimetallsalze, wie Lithium-, Natrium- oder Kaliumsalze, hergestellt und isoliert und in Form ihrer Salze zum Färben verwendet.

Ebenso können die in den nachfolgenden Beispielen, insbesondere Tabellenbeispielen, in Form der freien Säure genannten Ausgangsverbindungen und Komponenten als solche oder in Form ihrer Salze, vorzugsweise Alkalimetallsalze, in die Synthese eingesetzt werden.

Die für die erfindungsgemäßen Verbindungen angegebenen Absorptionsmaxima (λₘₐₓ) im sichtbaren Bereich wurden anhand derer Alkalimetallsalze in wäßriger Lösung ermittelt. In den Tabellenbeispielen sind die λₘₐₓ-Werte bei der Farbtonangabe in Klammern gesetzt; die Wellenlängenangabe bezieht sich auf nm.

### Beispiel 1

Eine Lösung von 25,3 Teilen Anilin-2,5-disulfonsäure in einem Gemisch 15,4 Teilen konzentrierter Schwefelsäure und 500 Teilen Wasser diazotiert man bei 5 bis 10°C mit 17,3 Teilen einer 40%igen wäßrigen Natriumnitritlösung. Überschüssige salpetrige Säure wird wie üblich mit Amidosulfonsäure zerstört. Danach gibt man 21,8 Teile 1-Aminonaphthalin-8-sulfonsäure hinzu und führt die Kupplungsreaktion ohne weitere Kühlung bei einem pH-Wert von 2 bis 3 durch. Die erhaltene Aminoazoverbindung wird innerhalb von etwa zwei bis drei Stunden mit 37,4 Teilen 3-(β-Chlorethylsuflonyl)-benzoylchlorid unter Einhaltung einer Temperatur von 15 bis 30°C und einem pH-Wert von 6,5 bis 7,5 unter gutem Rühren versetzt. Danach wird die erhaltene Lösung mit der erfindungsgemäßen Azoverbindung geklärt und aus dem Filtrat die Azoverbindung in üblicher Weise durch Aussalzen mit Natriumchlorid oder Kaliumchlorid oder durch Eindampfen unter reduziertem Druck oder durch Sprühtrocknung isoliert.

Man erhält ein gut wasserlösliches elektrolythaltiges (Natrium- oder Kaliumchloridhaltiges) Pulver des Alkalimetallsalzes (Natrium- oder Kaliumsalzes) der Verbindung der Formel 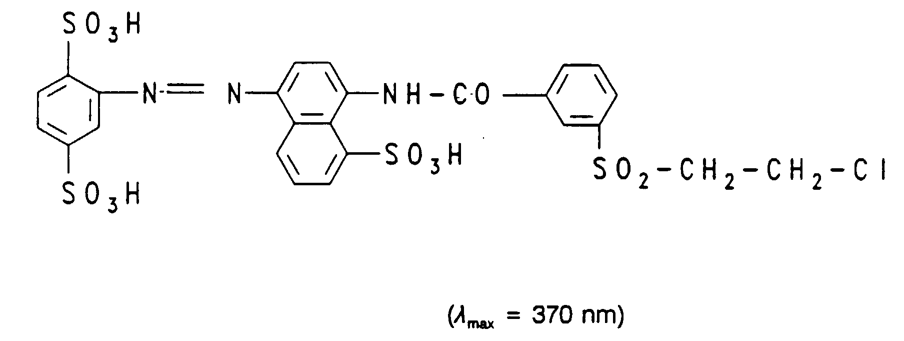 die sehr gute faserreaktive Farbstoffeigenschaften besitzt. Nach den für faserreaktive Farbstoffe üblichen Applikations- und Fixierverfahren liefert die erfindungsgemäße Azoverbindung auf den in Beschreibung genannten Fasermaterialien, wie insbesondere Cellulosefasermaterialien, farbstarke, klare, neutral gelbe Färbungen und Drucke mit guten Echtheitseigenschaften, von denen insbesondere die Licht-, Wasch-, Chlor-, Schweiß-, Naßlicht- und Schweißlichtechtheiten hervorgehoben werden können. Die erfindungsgemäße Azoverbindung hat ein gutes Aufbauvermögen. Die mit ihr erhältlichen Drucke zeigen einen klaren Weißfond, und nicht fixierte Anteile der Verbindung sind gut auswaschbar. Mit Reservierungsmitteln imprägnierte Gewebeteile werden von der erfindungsgemäßen Azoverbindung nicht angefärbt, so daß sie sehr gut zur Herstellung von klaren, scharfen Mustern im Reservedruck eingesetzt werden kann. Färbungen des Farbstoffes lassen sich darüber hinaus mit den technisch üblichen Ätzmitteln weiß ätzen.

### Beispiel 2

Ein Gemisch einer neutralen Lösung von 38,3 Teilen 2-Aminonaphthalin-4,6,8-trisulfonsäure in 500 Teilen Wasser und 17,4 Teilen einer 40%igen wäßrigen Natriumnitritlösung werden langsam in ein Gemisch aus 15,5 Teilen konzentrierter Schwefelsäure und 200 Teilen Eis eingerührt. Man rührt noch eine Stunde nach, entfernt überschüssige salpetrige Säure und gibt zu der so erhaltenen Diazoniumsalzlösung 14,5 Teile 3-Amino-phenylharnstoff hinzu. Die Kupplungsreaktion wird bei einem pH-Wert von 2 und bei einer Temperatur von 15 bis 20°C durchgeführt. Danach wird der Ansatz auf einen pH-Wert von 6 bis 7 gestellt und innerhalb von etwa zwei Stunden mit einer Lösung von 33,7 Teilen 4-(β-Chlorethylsulfonyl-methyl)-benzoylchlorid in 100 Vol.-Teilen Toluol versetzt. Nach Beendigung der Acylierungsreaktion wird der pH-Wert des Ansatzes auf 4 gestellt, zum Sieden erhitzt und das Toluol abdestilliert. Die Lösung wird geklärt und die erhaltene erfindungsgemäße Azoverbindung durch Eindampfen des Filtrates isoliert.

Man erhält die erfindungsgemäße Verbindung der, in Form der freien Säure geschrieben, Formel 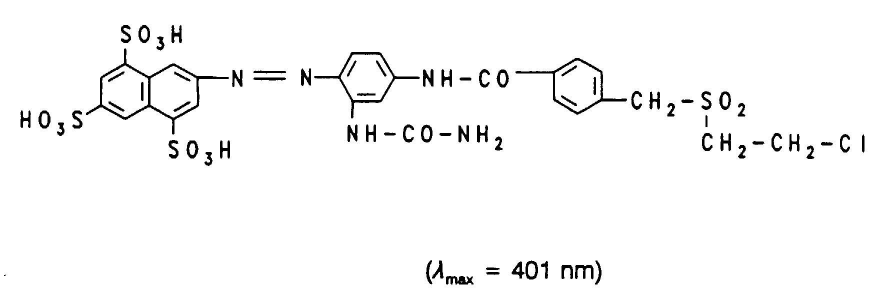 als Alkalimetallsalz (Natriumsalz) in Form eines rotbraunen elektrolythaltigen (vorwiegend natriumchloridhaltigen) Pulvers.

Die erfindungsgemäße Azoverbindung liefert nach den in der Technik üblichen Anwendungsverfahren für faserreaktive Farbstoffe farbstarke rotstichig gelbe Färbungen und Drucke mit guten Echtheitseigenschaften, von denen insbesondere die Licht-, Wasch-, Chlor-, Schweiß-, Naßlicht- und Schweißlichtechtheiten hervorgehoben werden können. Die erfindungsgemäße Azoverbindung hat ein gutes Aufbauvermögen. Die mit ihr erhältlichen Drucke zeigen einen klaren Weißfond, und nicht fixierte Anteile der Verbindung sind gut auswaschbar. Mit Reservierungsmitteln imprägnierte Gewebeteile werden von der erfindungsgemäßen Azoverbindung nicht angefärbt, so daß sie sehr gut zur Herstellung von klaren, scharfen Mustern im Reservedruck eingesetzt werden kann. Färbungen des Farbstoffes lassen sich darüber hinaus mit den technisch üblichen Ätzmitteln weiß ätzen.

### Beispiel 3

Man diazotiert 20,3 Teile 2-Aminonaphthalin-4,8-disulfonsäure analog den Angaben des Beispieles 2 zur Diazotierung des dort eingesetzten Naphthylamins und setzt zu der erhaltenen Diazoniumsalzlösung 10,7 Teile 3-Methyl-anilin hinzu. Man führt die Kupplungsreatkion bei einem pH-Wert von 2 und einer Temperatur von 15 bis 20°C durch und gibt anschließend, nach Einstellen des Reaktionsansatzes auf einen pH-Wert von 5, während etwa drei Stunden 43,8 Teile 2-(β-Chlorethylsulfonyl)-propionylchlorid unter Einhaltung eines pH-Bereiches von 6 bis 9 und einer Temperatur von 10 bis 20°C hinzu. Nach Beendigung der Acylierungsreaktion stellt man den Ansatz auf einen pH-Wert von 3,5 bis 4, klärt ihn und isoliert die erfindungsgemäße Azoverbindung durch Sprühtrocknung.

Die erfindungsgemäße Azoverbindung, die, in Form der freien Säure geschrieben, die Formel 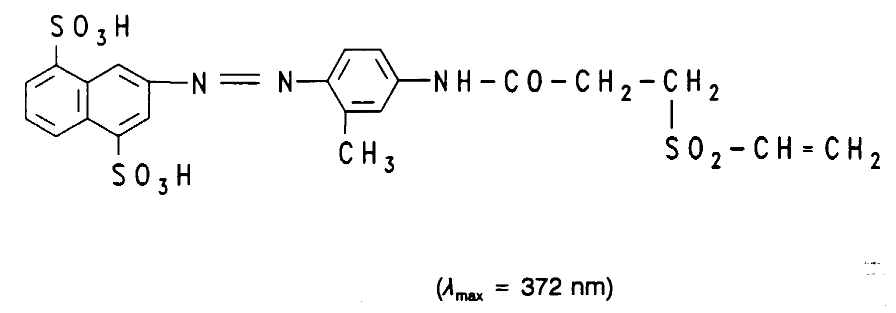 besitzt, zeigt sehr gute faserreaktive Farbstoffeigenschaften und liefert nach den in der Technik üblichen Färbeweisen für faserreaktive Farbstoffe farbstarke gelbe Drucke mit guten Echtheitseigenschaften, von denen insbesondere die Licht-, Wasch-, Chlor-, Schweiß-, Naßlicht- und Schweißlichtechtheiten hervorgehoben werden können. Die erfindungsgemäße Azoverbindung hat ein gutes Aufbauvermögen. Die mit ihr erhältlichen Drucke zeigen einen klaren Weißfond, und nicht fixierte Anteile der Verbindung sind gut auswaschbar. Mit Reservierungsmitteln imprägnierte Gewebeteile werden von der erfindungsgemäßen Azoverbindung nicht angefärbt, so daß sie sehr gut zur Herstellung von klaren, scharfen Mustern im Reservedruck eingesetzt werden kann. Färbungen des Farbstoffes lassen sich darüber hinaus mit den technisch üblichen Ätzmitteln weiß ätzen.

### Beispiel 4

Zur Herstellung einer erfindungsgemäßen Azoverbindung verfährt man gemäß der Verfahrensweise des Beispieles 2 der Diazotierung von 38,3 Teilen 2-Aminonaphthalin-4,6,8-trisulfonsäure, Kupplung des erhaltenen Diazoniumsalzes mit 14,5 Teilen N-Methyl-anilin und Acylierung der erhaltenen Amino-Azoverbindung mit 32 Teilen 3-(β-Chlorethylsulfonyl)-benzoylchlorid. Man isoliert die erhaltene erfindungsgemäße Azoverbindung in üblicher Weise. Sie besitzt, in Form der freien Säure geschrieben, die Konstitution der Formel 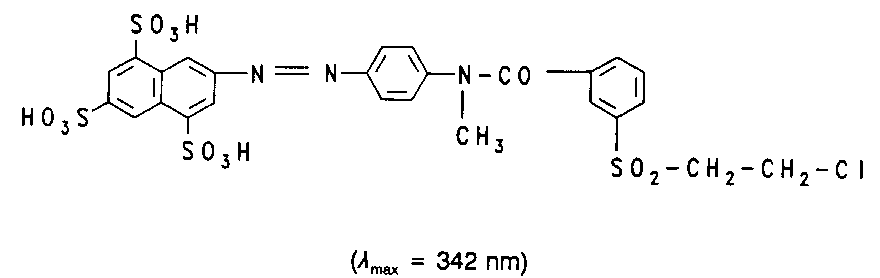 und liefert auf den in der Beschreibung genannten Materialien, wie insbesondere Cellulosefasermaterialien, beispielsweise Baumwolle, farbstarke gelbe Färbungen und Drucke mit guten Echtheitseigenschaften, von denen insbesondere die Licht-, Wasch-, Chlor-, Schweiß-, Naßlicht- und Schweißlichtechtheiten hervorgehoben werden können. Die erfindungsgemäße Azoverbindung hat ein gutes Aufbauvermögen. Die mit ihr erhältlichen Drucke zeigen einen klaren Weißfond, und nicht fixierte Anteile der Verbindung sind gut auswaschbar. Mit Reservierungsmitteln imprägnierte Gewebeteile werden von der erfindungsgemäßen Azoverbindung nicht angefärbt, so daß sie sehr gut zur Herstellung von klaren, scharfen Mustern im Reservedruck eingesetzt werden kann. Färbungen des Farbstoffes lassen sich darüber hinaus mit den technisch üblichen Ätzmitteln weiß ätzen.

### Beispiele 5 bis 65

In den nachfolgenden Tabellenbeispielen sind weitere erfindungsgemäße Azoverbindungen mit Hilfe der Komponenten der allgemeinen Formel (1A)

D - N = N - (4) K - (1) N(R) - CO - A - W - SO₂-X (1A)

beschrieben. Sie lassen sich in erfindungsgemäße Weise, beispielsweise nach einem der obigen Ausführungsbeispiele, mittels ihrer aus dem jeweiligen Tabellenbeispiel in Verbindung mit der Formel (1A) ersichtlichen Komponenten (der Diazokomponente D-NH₂, der Kupplungskomponente H-K-N(R)H und dem Säurehalogenid entsprechend der allgemeinen Formel (3)) herstellen. Sie besitzen gute faserreaktive Farbstoffeigenschaften und liefern auf den in der Beschreibung genannten Materialien, wie insbesondere Cellulosefasermaterialien, nach den für faserreaktive Farbstoffe üblichen Applikations- und Fixiermethoden farbstarke, echte Färbungen und Drucke in dem in dem jeweiligen Tabellenbeispiel für Färbungen auf Baumwolle angegebenen Farbton. Sind darüber hinaus sehr gut sowohl für den Reservedruck als auch für den Ätzdruck geeignet.

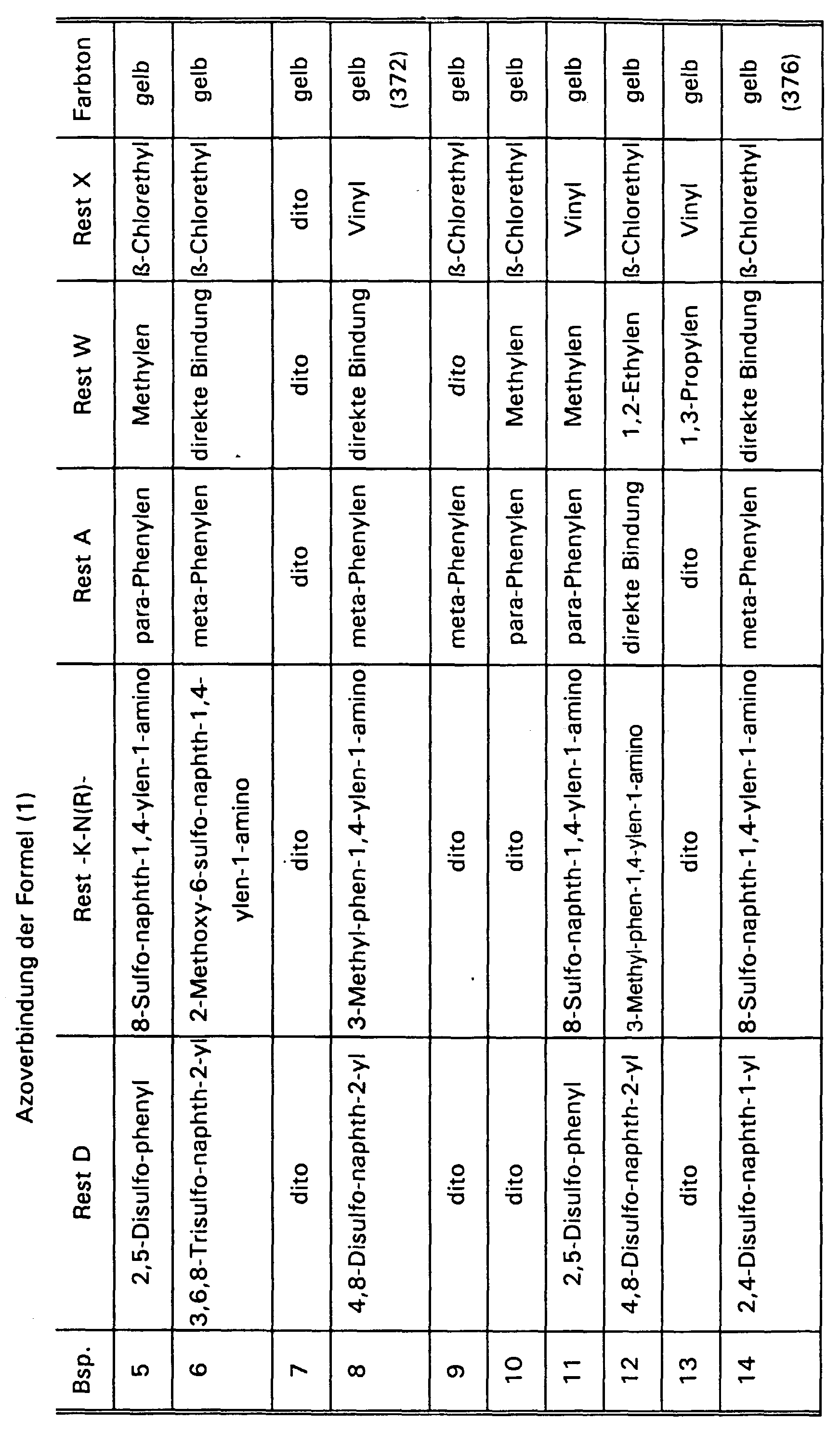

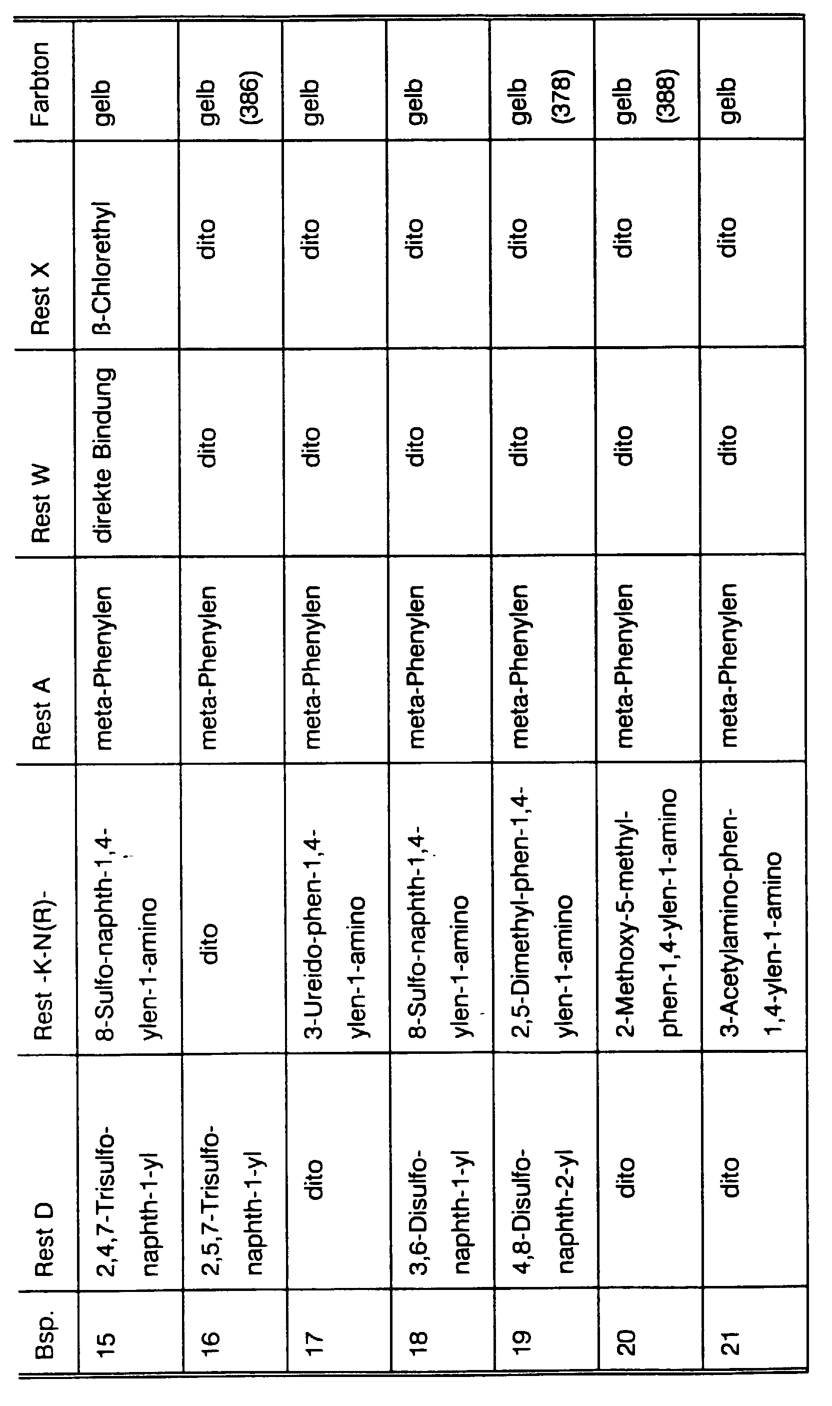

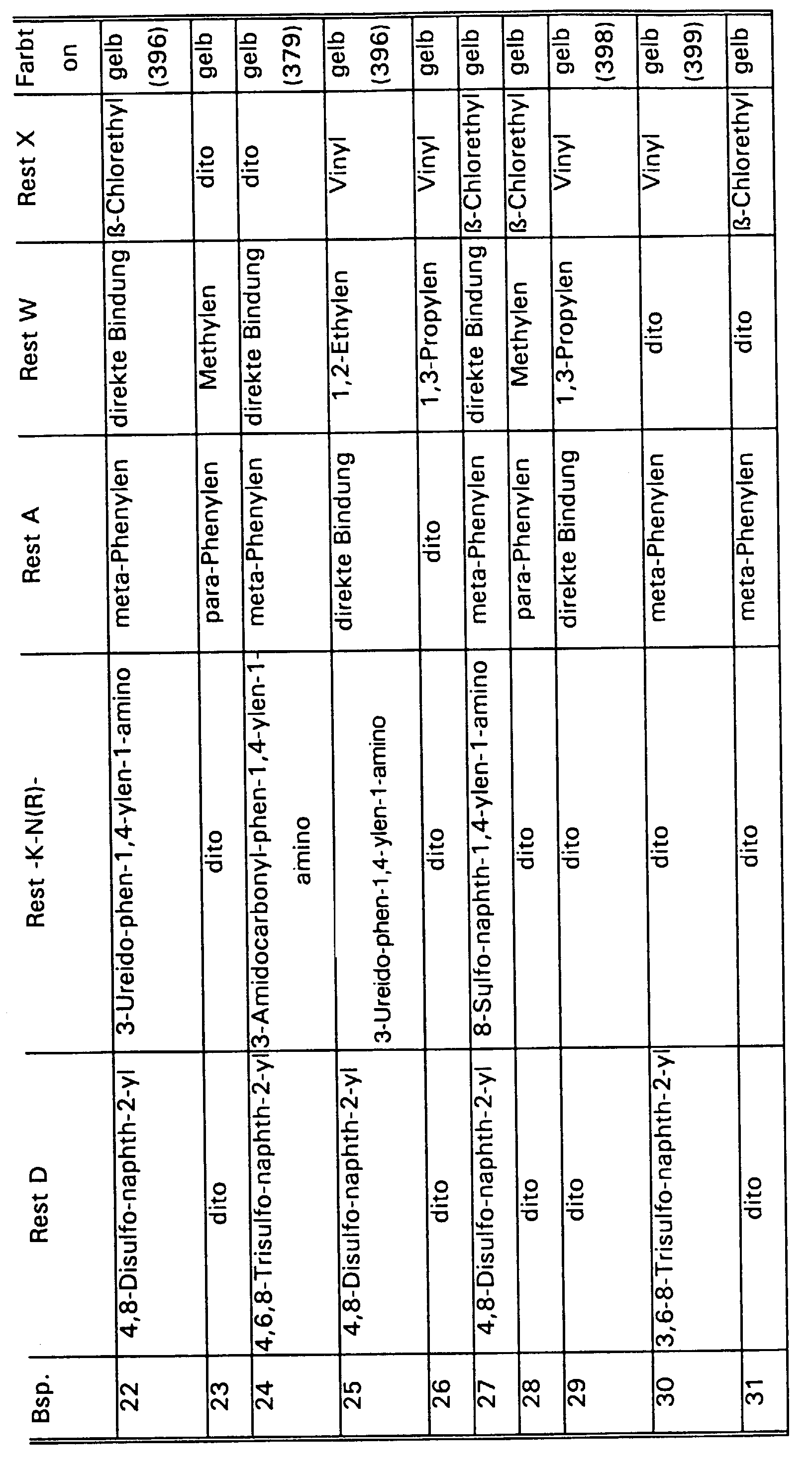

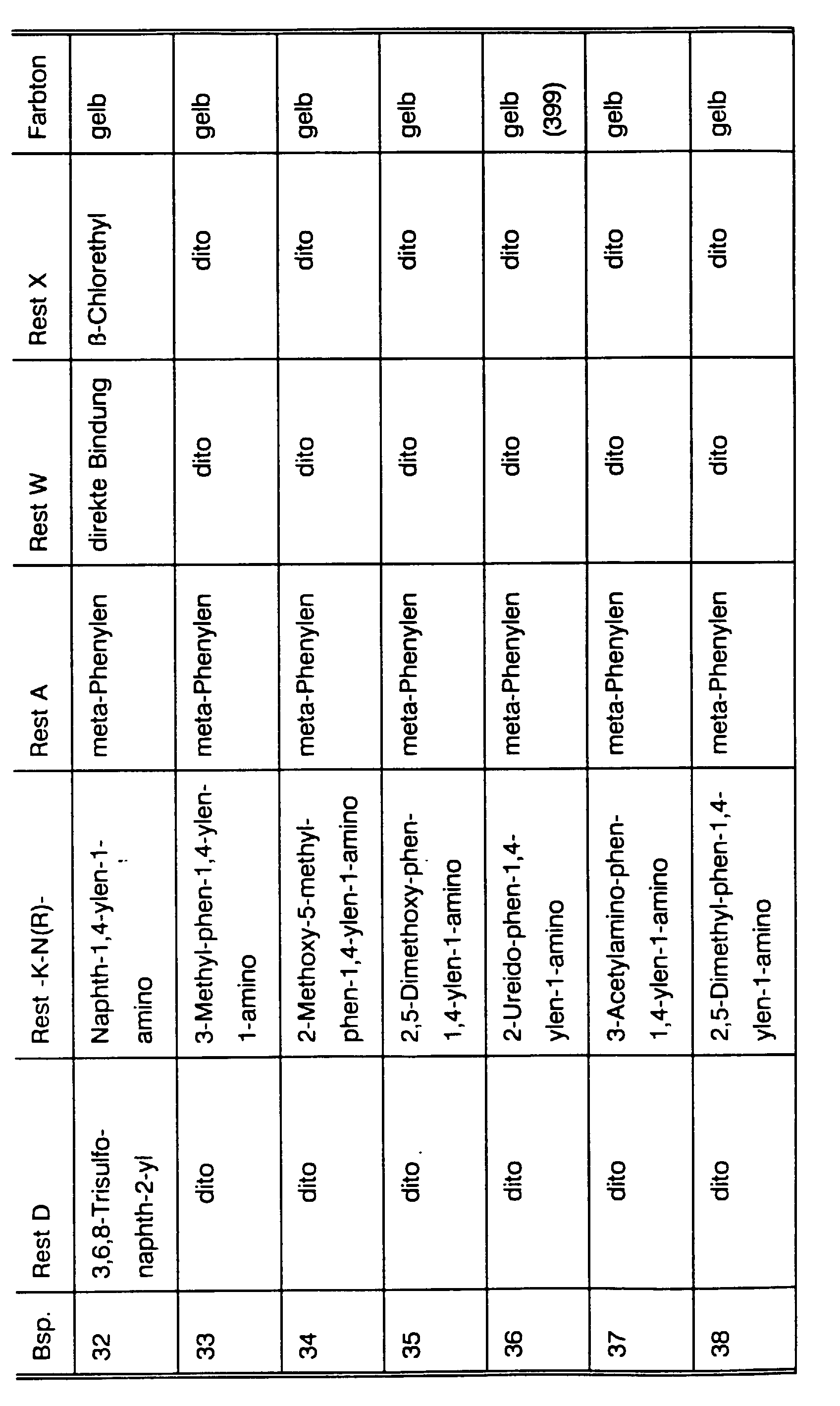

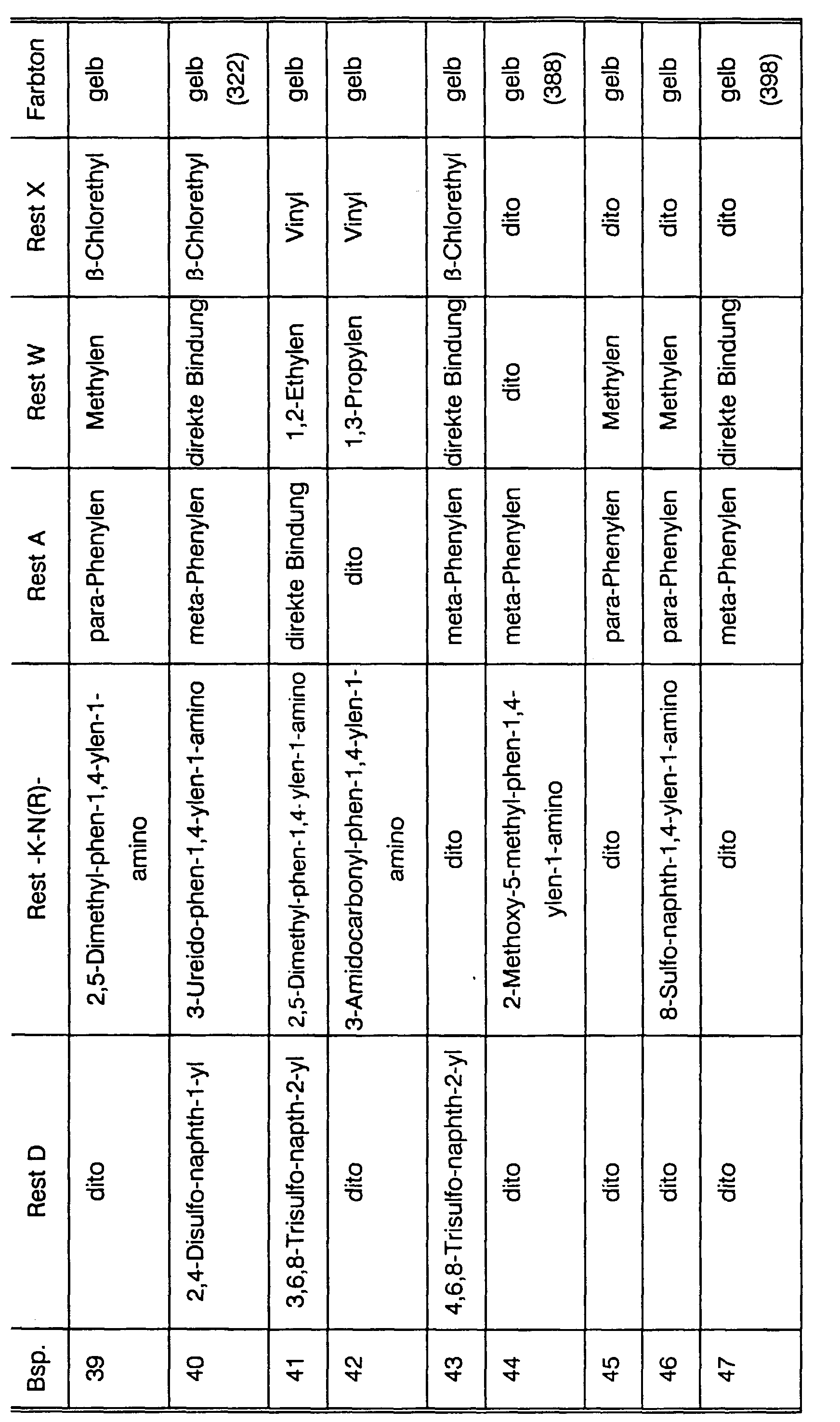

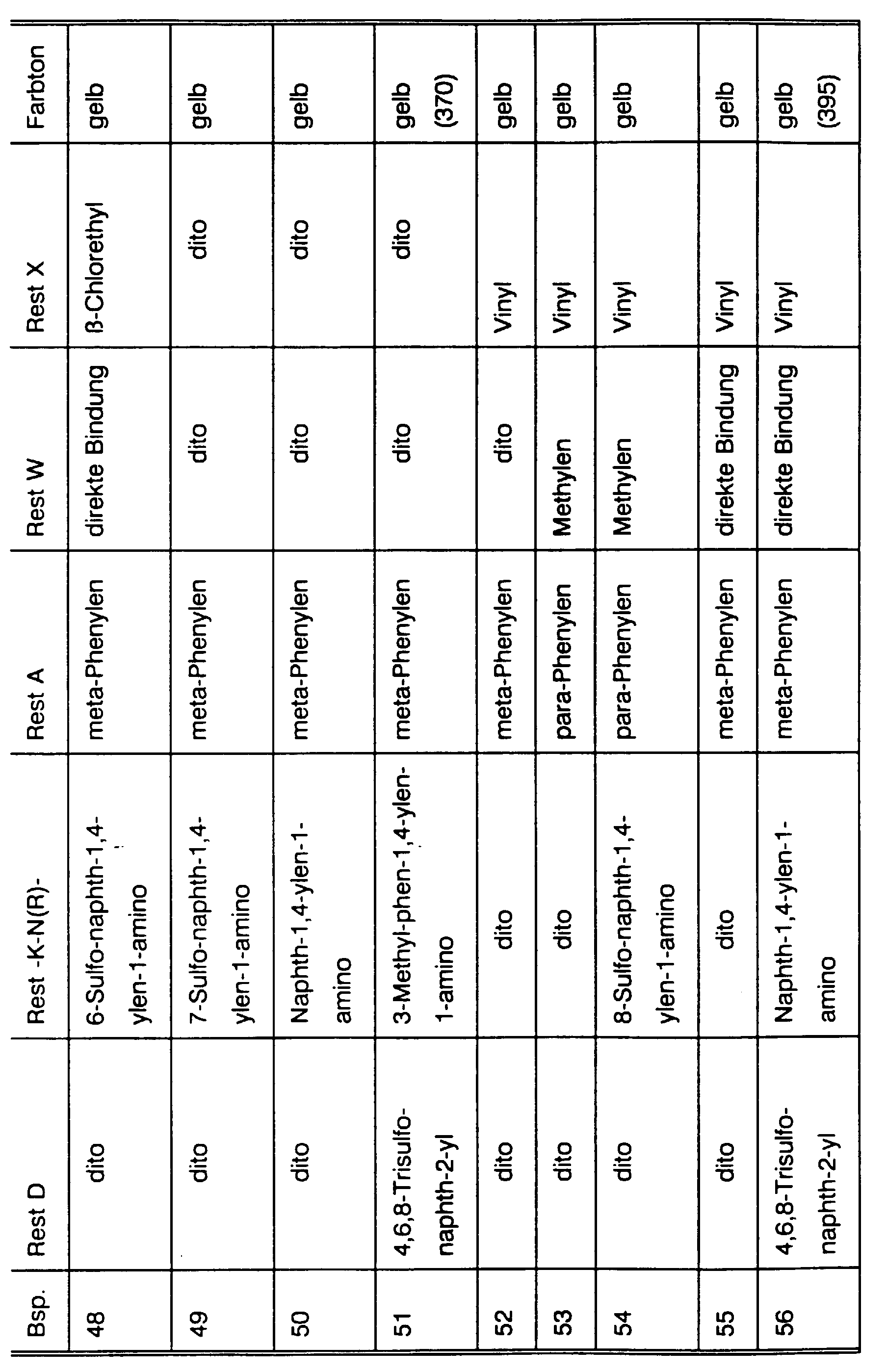

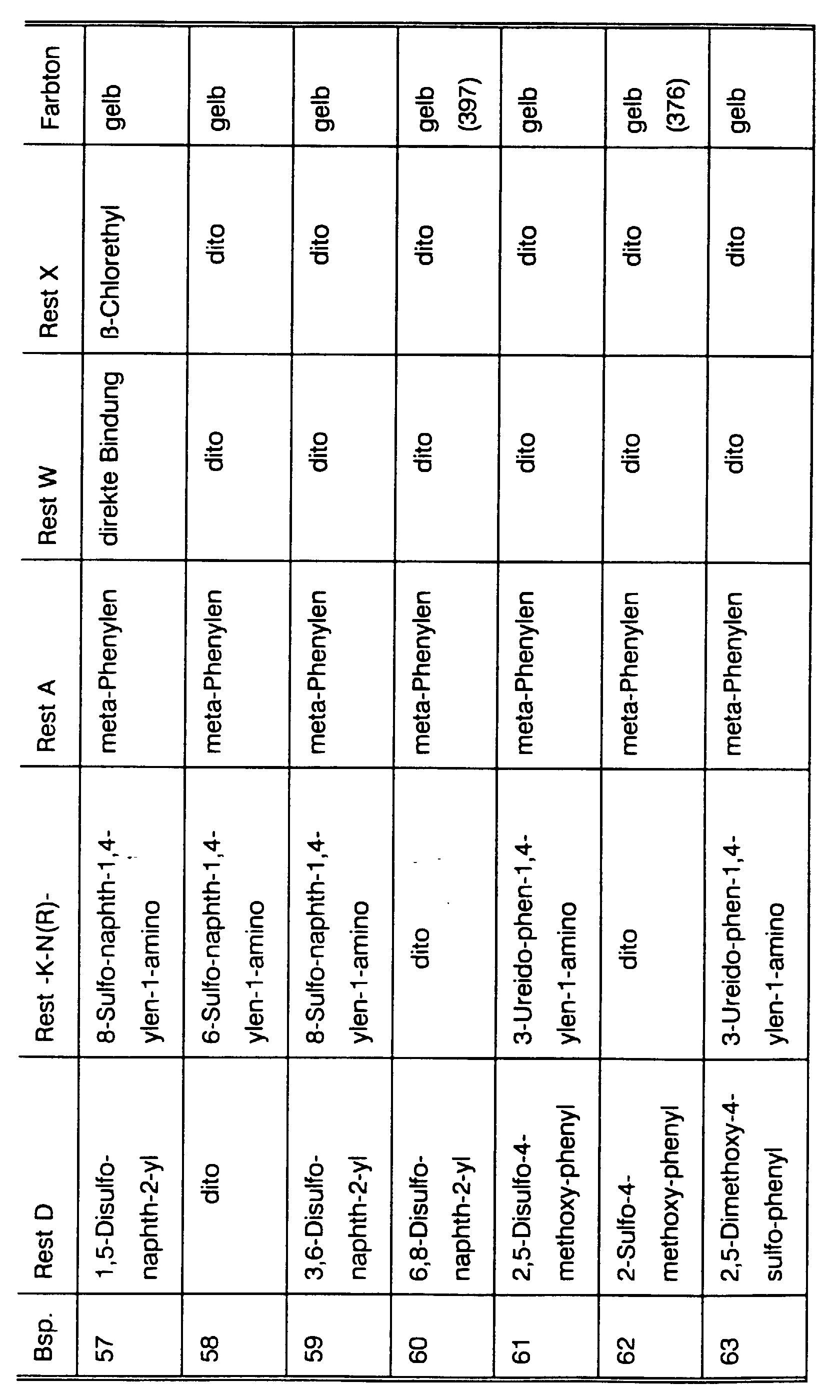

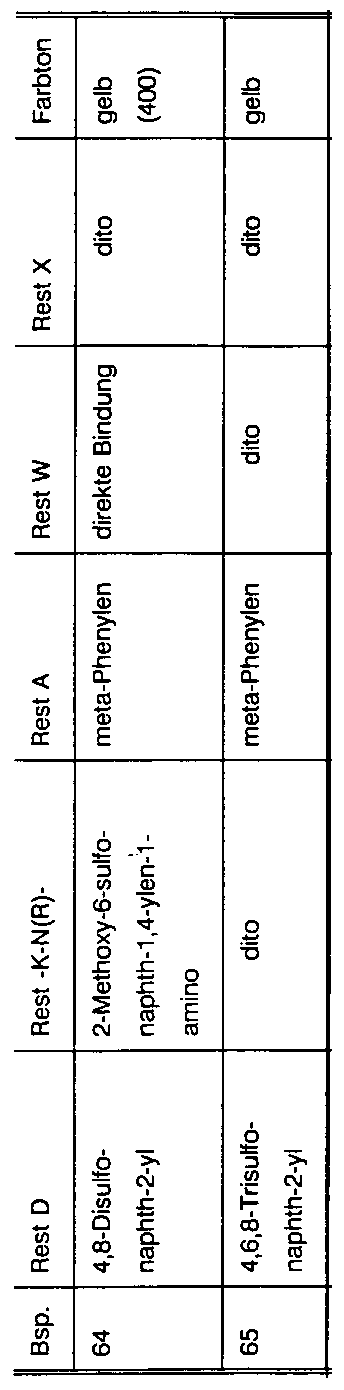

## Patentansprüche

1. Monoazoverbindung entsprechend der allgemeinen Formel (1) 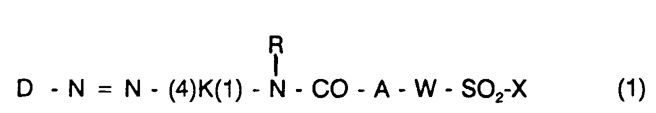 in welcher bedeuten:
D ist Monosulfophenyl oder Disulfophenyl, die beide durch 1 oder 2 Substituenten aus der Gruppe Alkyl von 1 bis 4 C-Atomen, Alkoxy von 1 bis 4 C-Atomen, Halogen, Carboxy und Alkanoylamino von 2 bis 5 C-Atomen substituiert sein können, oder ist Mono-, Di- oder Trisulfonaphthyl;
K ist als Rest einer Kupplungskomponente der para-Phenylenrest, der unsubstituiert oder durch 1 oder 2 Substituenten aus der Gruppe Alkyl von 1 bis 4 C-Atomen, Alkoxy von 1 bis 4 C-Atomen, Halogen, Alkanoylamino von 2 bis 5 C-Atomen, Ureido, Carbamoyl und Sulfo substituiert ist, oder ist der 1,4-Naphthylen-Rest, der durch 1 oder 2 Sulfogruppen und/oder 1 Alkoxygruppe von 1 bis 4 C-Atomen substituiert sein kann;
R ist Wasserstoff oder Alkyl von 1 bis 4 C-tomen, bevorzugt Wasserstoff;
A ist Phenylen oder eine direkte Bindung;
W ist eine direkte Bindung oder Alkylen von 1 bis 4 C-Atomen, wobei A und W nicht gleichzeitig eine direkte Bindung sind;
X ist Vinyl oder ist Ethyl, das in β-Stellung durch einen Substituenten substituiert ist, der durch Alkali unter Bildung der Vinylgruppe eliminierbar ist.

2. Monoazoverbindungen nach Anspruch 1, dadurch gekennzeichnet, daß D ein Monosulfo- oder Disulfo-naphth-2-yl-Rest ist.

3. Monoazoverbindung nach Anspruch 1 oder 2, dadurch gekennzeichnet, daß der Rest -(4)K(1)-N(R)- der 3-Ureido-phen-1,4-ylen-1-amino-Rest ist.

4. Monoazoverbindung nach Anspruch 1 oder 2, dadurch gekennzeichnet, daß der Rest -(4)K(1)-N(R)- ein monosulfosubstituierter Naphth-1,4-ylen-1-amino-Rest ist.

5. Monoazoverbindung nach mindestens einem der Ansprüche 1 bis 4, dadurch gekennzeichnet, daß A der 1,3-Phenylen-Rest ist.

6. Monoazoverbindung nach mindestens einem der Ansprüche 1 bis 5, dadurch gekennzeichnet, daß X Vinyl, β-Sulfatoethyl oder β-Chlorethyl ist.

7. Monoazoverbindung nach mindestens einem der Ansprüche 1 bis 5, dadurch gekennzeichnet, daß X β-Chlorethyl ist.

8. Verfahren zur Herstellung einer Monoazoverbindung von Anspruch 1, dadurch gekennzeichnet, daß man eine Amino-Azoverbindung der allgemeinen Formel (2)
D - N = N - K - N(R) - H (2)
in welcher D, K und R die in Anspruch 1 genannten Bedeutungen haben, mit einer Verbindung der allgemeinen Formel (3)
Hal - CO - A - W - SO₂-X (3)
in welcher Hal für Brom oder bevorzugt Chlor steht und A, W und X die in Anspruch 1 gennannten Bedeutungen haben, bei einer Temperatur zwischen 0 und 40°C und bei einem pH-Wert zwischen 1 und 9 umsetzt.

9. Verwendung eine Monoazoverbindung von Anspruch 1 zum Färben (einschließlich Bedrucken) von hydroxy- und/oder carbonamidgruppenhaltigem Material, insbesondere Fasermaterial.

10. Verfahren zum Färben (einschließlich Bedrucken) von hydroxy- und/oder carbonamidgruppenhaltigem Material, vorzugsweise Fasermaterial, bei welchem man einen Farbstoff auf das Material aufbringt oder in das Material einbringt und den Farbstoff mittels Wärme oder mit Hilfe eines alkalisch wirkenden Mittels oder durch beide Maßnahmen auf bzw. in dem Material fixiert, dadurch gekennzeichnet, daß man als Farbstoff eine Monoazoverbindung von Anspruch 1 verwendet.

## Claims

1. A monoazo compound conforming to the formula (1) 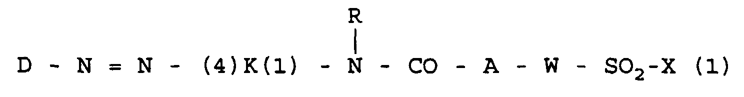 where:
D is monosulfophenyl or disulfophenyl, which may each be substituted by 1 or 2 substituents selected from the group consisting of alkyl of 1 to 4 carbon atoms, alkoxy of 1 to 4 carbon atoms, halogen, carboxyl and alkanoylamino of 2 to 5 carbon atoms, or is mono-, di- or trisulfonaphthyl;
K, the radical of a coupling component, is paraphenylene, which is unsubstituted or substituted by 1 or 2 substituents selected from the group consisting of alkyl of 1 to 4 carbon atoms, alkoxy of 1 to 4 carbon atoms, halogen, alkanoylamino of 2 to 5 carbon atoms, ureido, carbamoyl and sulfo, or is 1,4-naphthylene, which may be substituted by 1 or 2 sulfo groups and/or 1 alkoxy group of 1 to 4 carbon atoms;
R is hydrogen or alkyl of 1 to 4 carbon atoms, preferably hydrogen;
A is phenylene or a direct bond;
W is a direct bond or alkylene of 1 to 4 carbon atoms, where A and W are not both a direct bond;
X is vinyl or is ethyl substituted in the β-position by a substituent eliminable by alkali to form the vinyl group.

2. Monoazo compounds as claimed in claim 1, wherein D is monosulfo- or disulfo-naphth-2-yl.

3. A monoazo compound as claimed in claim 1 or 2, wherein the radical -(4)K(1)-N(R)- is 3-ureidophen-1,4-ylen-1-amino.

4. A monoazo compound as claimed in claim 1 or 2, wherein the radical -(4)K(1)-N(R)- is monosulfo-substituted naphth-1,4-ylen-1-amino.

5. A monoazo compound as claimed in at least one of claims 1 to 4, wherein A is 1,3-phenylene.

6. A monoazo compound as claimed in at least one of claims 1 to 5, wherein X is vinyl, β-sulfatoethyl or β-chloroethyl.

7. A monoazo compound as claimed in at least one of claims 1 to 5, wherein X is β-chloroethyl.

8. A process for preparing a monoazo compound as claimed in claim 1, which comprises reacting an aminoazo compound of the formula (2)
D - N = N - K - N (R) - H (2)
where D, K and R are each as defined in claim 1 with a compound of the formula (3)
Hal - CO - A - W - SO₂-X (3)
where Hal is bromine or preferably chlorine and A, W and X are each as defined in claim 1 at a temperature between 0 and 40°C and at a pH between 1 and 9.

9. The use of a monoazo compound as claimed in claim 1 for dyeing (including printing) hydroxyl- and/or carboxamido-containing material, especially fiber material.

10. A process for dyeing (including printing) hydroxyl- and/or carboxamido-containing material, preferably fiber material, in which a dye is applied to or introduced into the material and fixed thereon or therein by means of heat or with the aid of an alkaline agent or by both measures, which comprises using a monoazo compound as claimed in claim 1 as dye.

## Revendications

1. Composé monoazoïque correspondant à la formule générale (1) 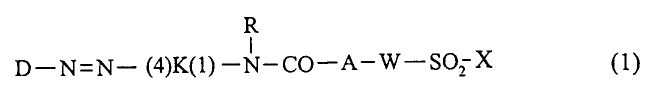 dans laquelle
D représente un reste monosulfophényle ou un reste disulfophényle, qui peuvent tous les deux être substitués par 1 ou 2 substituants choisis dans le groupe constitué par les restes alkyle de 1 à 4 atomes de carbone, alcoxy de 1 à 4 atomes de carbone, halogéno, carboxy et alcanoylamino de 2 à 5 atomes de carbone, ou D est un reste mono-, di- ou trisulfonaphtyle;
K représente, en tant que reste d'un copulant, un reste p-phénylène non substitué ou substitué par 1 ou 2 substituants choisis dans le groupe constitué par les restes alkyle de 1 à 4 atomes de carbone, alcoxy de 1 à 4 atomes de carbone, halogéno, alcanoylamino de 2 à 5 atomes de carbone, uréido, carbamoyle et sulfo, ou K est un reste 1,4-naphtylène pouvant être substitué par 1 ou 2 groupes sulfo et/ou 1 groupe alcoxy de 1 à 4 atomes de carbone;
R est un atome d'hydrogène ou un reste alkyle de 1 à 4 atomes de carbone, de préférence un atome d'hydrogène;
A est un reste phénylène ou une liaison directe;
W est une liaison directe ou un reste alkylène de 1 à 4 atomes de carbone, A et W ne devant cependant pas être une liaison directe en même temps;
X est un reste vinyle, ou un reste éthyle substitué en position β par un substituant éliminable en milieu basique avec formation du groupe vinyle.

2. Composé monoazoïque selon la revendication 1, caractérisé en ce que D est un reste monosulfo- ou disulfonapht-2-yle.

3. Composé monoazoïque selon la revendication 1 ou 2, caractérisé en ce que le reste -(4)K(1)-N(R)- est le reste 3-uréidophén-1,4-ylène-1-amino.

4. Composé monoazoïque selon la revendication 1 ou 2, caractérisé en ce que le reste -(4)K(1)-N(R)- est un reste napht-1,4 ylène-1-anlino monosubstitué par sulfo.

5. Composé monoazoïque selon l'une au moins des revendications 1 à 4, caractérisé en ce que A est le reste 1,3-phénylène.

6. Composé monoazoïque selon l'une au moins des revendications 1 à 5, caractérisé en ce que X est un reste vinyle, β-sulfatoéthyle ou β-chloroéthyle.

7. Composé monoazoïque selon l'une au moins des revendications 1 à 5, caractérisé en ce que X est le reste β-chloroéthyle.

8. Procédé de préparation d'un composé monoazoïque selon la revendication 1, caractérisé en ce que l'on fait réagir un composé aminoazoïque de formule générale (2)
D - N = N - K - N(R) - H (2)
dans laquelle D, K et R ont les significations indiquées dans la revendication 1, avec un composé de formule générale (3)
Hal - CO - A - W - SO₂ - X (3)
dans laquelle Hal est un atome de brome ou de préférence de chlore et A, W et X ont les significations indiquées dans la revendication 1, à une température comprise entre 0 et 40°C, et à un pH compris entre 1 et 9.

9. Utilisation d'un composé monoazoïque selon la revendication 1 pour la teinture (y compris l'impression) d'une matière contenant des groupes hydroxy et/ou carboxamide, en particulier d'une matière fibreuse.

10. Procédé de teinture (y compris d'impression) d'une matière contenant des groupes hydroxy et/ou carboxamide, de préférence d'une matière fibreuse, selon lequel on applique un colorant sur la matière ou on incorpore un colorant dans la matière et on fixe le colorant sur ou dans la matière à l'aide de chaleur ou d'un agent basique ou par ces deux moyens, caractérisé en ce que l'on utilise comme colorant un composé monoazoïque selon la revendication 1.
